(19) Europäisches Patentamt
European Patent Office
Office européen des brevets

(11) Publication number: 0 136 754
A1

(12) EUROPEAN PATENT APPLICATION

(21) Application number: 84201271.8

(22) Date of filing: 04.09.84

(51) Int. Cl.⁴: C 07 D 417/04
A 01 N 43/82
//(C07D417/04, 285:08, 233:32)

(30) Priority: 06.09.83 US 529314

(43) Date of publication of application:
10.04.85 Bulletin 85/15

(84) Designated Contracting States:
AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Pilgram, Kurt Hans Gerhard
2607 Warwick Lane
Modesto California 95350(US)

(74) Representative: Hunter, Keith Roger Ian et al,
4 York Road
London SE1 7NA(GB)

(54) **Thiadiazole herbicides.**

(57) Herbicidally active compounds of the formula I:-

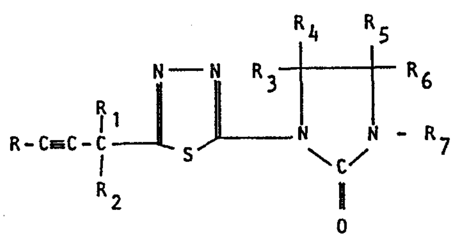

wherein R, $R_1$ and $R_2$ represent hydrogen or alkyl, $R_3$, $R_4$, $R_5$ and $R_6$ represent hydrogen, hydroxyl or oxo (subject to certain constraints), and $R_7$ represents alkyl, alkoxy or cycloalkyl; the preparation of such compounds and their use as herbicides and in herbicidal compositions.

EP 0 136 754 A1

K 3433 FF

# THIADIAZOLE HERBICIDES

This invention relates to certain thiadiazole imidazolidinone derivatives, the preparation of such compounds, herbicidal compositions containing them, and to their use in combating undesired plant growth.

Accordingly, the present invention provides compounds of the general formula I:-

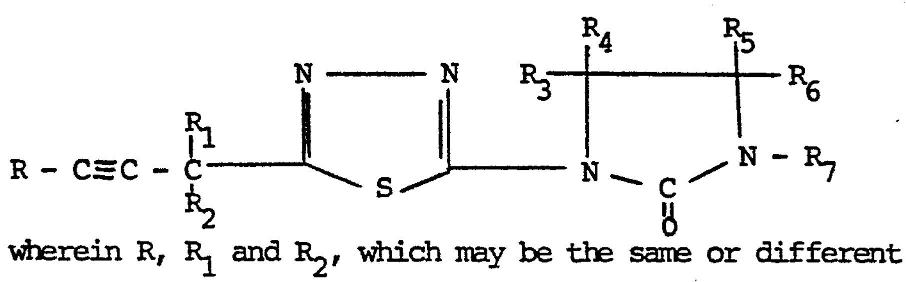

wherein R, $R_1$ and $R_2$, which may be the same or different, each independently represents a hydrogen atom or lower alkyl, preferably methyl or ethyl, group; $R_3$ is a hydrogen atom and $R_4$ is a hydroxyl group, or $R_3$ and $R_4$ together jointly represent an oxo(=O) group; $R_5$ is a hydrogen atom and $R_6$ is a hydrogen atom or hydroxyl group, or $R_5$ and $R_6$ together jointly represent an oxo(=O) group; and $R_7$ is a lower alkyl or alkoxy group, preferably methyl, or a cyclopropyl group.

The term "lower" alkyl and "lower" alkoxy is used herein to denote such groups containing up to 6, and in particular 1 to 3, carbon atoms. Preferred compounds are those wherein R represents a hydrogen atom and each of $R_1$, $R_2$ and $R_7$ represents a methyl group; also those wherein each of $R_3$, $R_5$ and $R_6$

represents a hydrogen atom and $R_4$ represents a methyl group.

The invention also provides a process for the preparation of compounds as defined above which comprises reacting a urea of formula III:

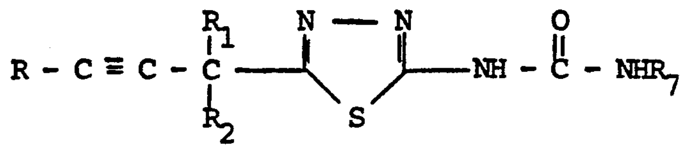

III

with a diketo compound of formula X-CO-CO-X, wherein both groups X represent a halogen, preferably chlorine, atom, or one X represents a hydrogen atom and the other represents a hydrogen atom or a hydroxyl group. The starting urea of formula III may be prepared from the precursor amine of formula II

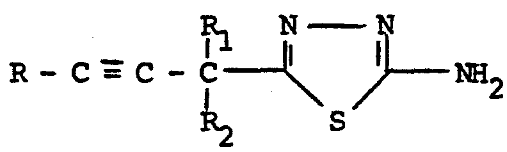

II

by conventional methods such as reaction with phosgene or an isocyanate of formula $R_7$-NCO.

The preparation of the preferred individual species of these amines (preferred because of the high phytotoxicity of the compounds of Formula I prepared therefrom), wherein R is hydrogen, and $R^1$ and $R^2$ each is methyl, is described in Example 1, hereinafter. Other individual species of these amines can be prepared by analogous methods. The article by M.A. Schexnayder and P.S. Engel, J. Am. Chem. Soc., 1975, 97, 4825-4836, discloses 2,2-dimethyl-3-butynoic acid, the precursor for the preferred amine and two methods for its preparation. Other needed precursors can be prepared by those methods.

For convenience in the more detailed description of the alternative process variants, the compounds of the invention may be subdivided into four subclasses as follows:

A. The imidazolidinetriones: $R^3$ and $R^4$ together are oxo, $R^5$ and $R^6$ together are oxo.

B. The hydroxyimidazolidinones:

1. $R^3$ is hydrogen, $R^4$ is hydroxy, $R^5$ and $R^6$ together are oxo;
2. $R^5$ is hydrogen, $R^6$ is hydroxy, $R^3$ and $R^4$ together are oxo.

C. The dihydroxyimidazolidinones: each of $R^3$ and $R^5$ is hydrogen, each of $R^4$ and $R^6$ is hydroxy;

D. The hydroxyimidazolidinones: each of $R^3$, $R^5$ and $R^6$ is hydrogen, $R^4$ is hydroxy.

Compounds of Subclass A can be prepared by treating the appropriate urea (of Formula III) with oxalyl chloride in the presence of an inert solvent, at a moderately elevated temperature. The reaction proceeds according to the equation:

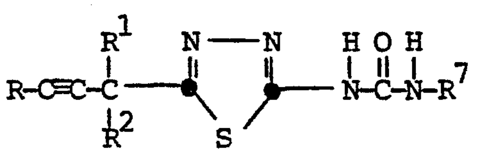 (III) + 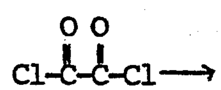

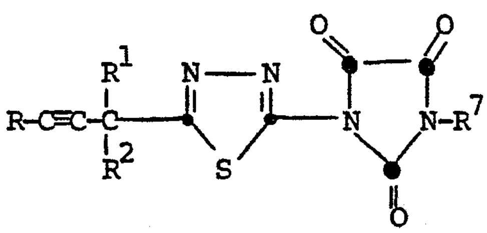 (IV) + 2HCl

Treatment of the urea with oxalyl chloride is conveniently conducted by adding the chloride to a stirred solution of the urea in an inert solvent such as toluene and refluxing the mixture for a sufficient time to enable the reaction to go to completion. The hydrogen chloride by-product is removed as a gas as it is evolved. Workup of the reaction mixture, isolation of the product and its purification are effected by conventional techniques, as illustrated in Example 1, hereinafter.

Compounds of Subclass B can be prepared by treating the appropriate urea (III) with glyoxylic acid (conveniently as the hydrate) in the presence of an inert solvent, at a moderately elevated temperature, The reaction proceeds according to the equation:

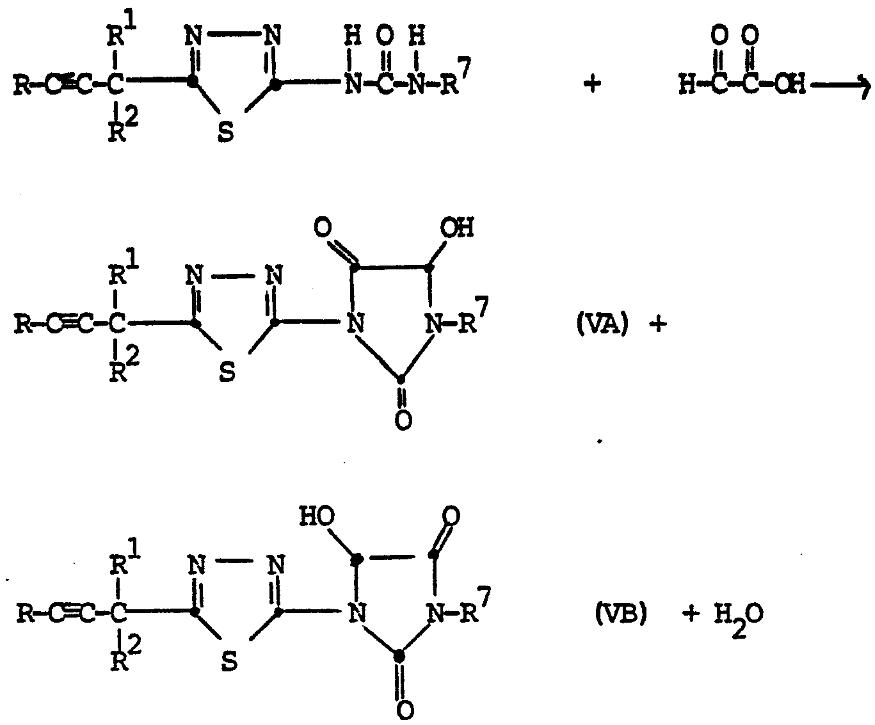

(VA) +

(VB) + $H_2O$

Treatment of the urea with glyoxylic acid is conveniently conducted by adding the acid to a stirred solution of the urea in an inert solvent such as methylene chloride and refluxing the mixture for a sufficient time to enable the reaction to go to completion. The water by-product preferably is removed as a vapour as it evolves. Workup of the reaction mixture, isolation of the product and its purification are effected by conventional techniques, as illustrated in Example 5, hereinafter.

Compounds of Subclass C can be prepared by treating the appropriate urea (III) with an aqueous solution of glyoxal at a moderately elevated temperature. The reaction proceeds according to the equation:

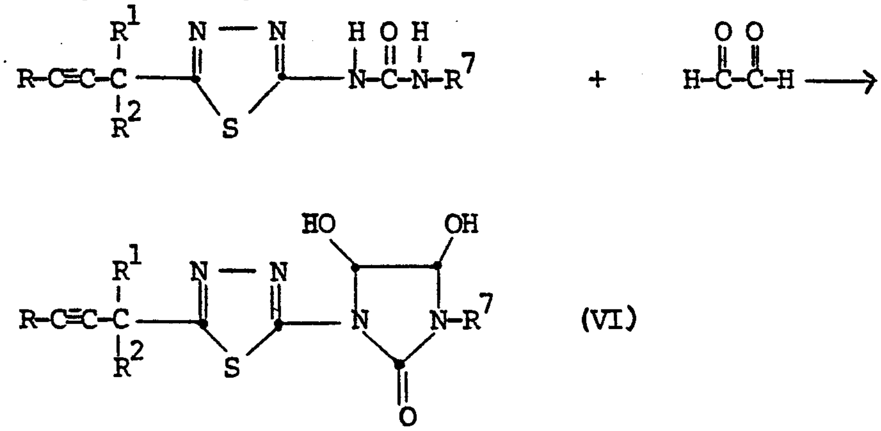

(VI)

Treatment of the urea with glyoxal is conveniently conducted by slowly adding the aquous solution of glyoxal to a refluxing solution of the urea in a solvent such as toluene, and removing water from the mixture azeotropically. Workup of the reaction mixture, isolation of the product and its purification are effected by conventional procedures, as illustrated in Example 4, hereinafter.

Compounds of Subclass D are prepared from the appropriate amine (II) by a three-step process in which

(a) the amine is treated with phosgene to form the isocyanate dimer:

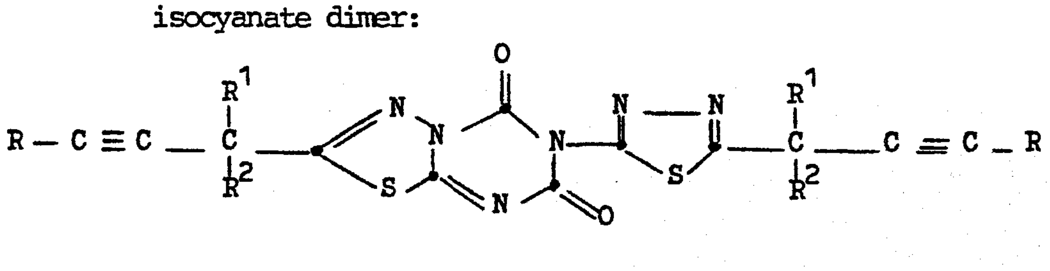

(b) the dimer is treated with a dimethyl acetal,

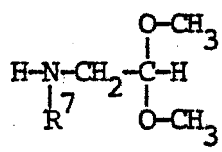

to form a compound of the formula

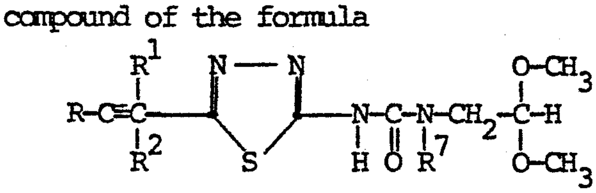

(c) which is heated in an acidic aqueous medium to form the compound of Subclass D, of the formula

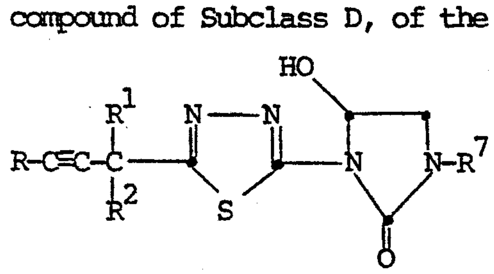

(VII)

The dimer can be prepared by treating the hydrochloride salt of the amine (II) with phosgene, use of the hydrochloride salt, rather than the amine per se, minimizing the formation of undesired symmetrical urea from the amine. The hydrochloride

salt is conveniently formed in situ by including hydrogen chloride in the reaction mixture: a solution of slurry of the amine in a solvent such as ethyl acetate is added to a solution of phosgene (two moles/mole of amine) in a solvent such as ethyl acetate also containing the hydrogen chloride. The resulting mixture is heated at a moderately elevated temperature for a time sufficient for the reaction to go to completion. Unreacted phosgene can be removed by purging the reaction mixture with nitrogen. The dimer can be isolated by evaporating the solvent. Then a solution of the dimer and the appropriate N-$R^7$-aminoacetaldehyde dimethylacetal in a solvent such as tetrahydrofuran is heated at a moderately elevated temperature to effect the reaction and stripped of solvent to give the crude product, which can be purified by recrystallization from, for example, a mixture of ether and hexane. That product is converted to a compound of Formula I by heating it at a moderate temperature in a dilute solution of an inert acid, such as hydrochloric acid. The desired product can be isolated by extracting the aqueous mixture with a suitable water-insoluble solvent, such as methylene chloride, and evaporating the solvent from the extract.

The preparation, isolation and testing of six typical individual species of the compounds of Formula I are described in the examples, following. The class of compounds is further illustrated and exemplified by the following further individual species, all of which are specifically contemplated in this invention. In the interest of brevity, in the identification of these species, they will be identified in terms of four subclasses:

| Species No. | R | $R^1$ | $R^2$ | $R^7$ |
|---|---|---|---|---|
| Subclass A: | | | | |
| 1 | H | methyl | methyl | cyclopropyl |
| 2 | " | " | " | methoxy |
| 3 | " | " | H | methyl |
| 4 | " | ethyl | " | " |
| 5 | methyl | methyl | methyl | " |

| Species No. | R | $R^1$ | $R^2$ | $R^7$ |
|---|---|---|---|---|
| Subclass B: | | | | |
| 6 | H | methyl | methyl | cyclopropane |
| 7 | H | " | " | methoxy |
| 8 | " | " | H | methyl |
| 9 | " | ethyl | " | " |
| 10 | methyl | methyl | methyl | " |
| Subclass C: | | | | |
| 11 | H | methyl | methyl | cyclopropyl |
| 12 | " | " | " | methoxy |
| 13 | " | " | H | methyl |
| 14 | " | ethyl | " | " |
| 15 | methyl | methyl | methyl | " |
| Subclass D: | | | | |
| 1 | H | methyl | methyl | cyclopropyl |
| 2 | " | " | " | methyl |
| 3 | " | ethyl | " | " |
| 4 | methyl | methyl | " | " |

Compounds of Formula I have been found to affect adversely the growth of plants, many of which are commonly considered as weeds, and therefore to be useful for controlling the growth of such unwanted plants. Compounds of Subclass D, Formula I, have been found to have selectivity with respect to some crop plants — i.e., they control weeds at dosages at which they do not significantly harm the crop plants. Compounds of Formula I appear to be effective when applied preemergence or preplant incorporated (applied to the soil before the seeds have sprouted) or when applied postemergence (applied to the foliage of the growing plant). They appear to be somewhat more effective when applied preemergence than when applied postemergence.

Accordingly, the invention includes a method of combating unwanted plants which comprises applying to the locus an effective amount of a compound of Formula I. In the cases where it is desired to control weeds in crop plantings, it is of

course preferable to employ the lowest dosage that will control the weeds, for this will minimize any possible deleterious effect of the compound upon the crop plants.

Protection of a locus or area from undesirable plants is effected by applying a compound of Formula I, ordinarily in a composition of one of the types described below, to soil in which the seeds of the unwanted plants are present, or to the foliage of the unwanted plants. The active compound, of course, is applied in an amount sufficient to exert the desired action. The amount of the compound of the invention to be used in combating undesired plants will naturally depend on the condition of the plants, the degree of activity desired, the formulation used, the mode of application, the climate, the season of the year, and other variables. Recommendations as to precise amounts are, therefore, not possible. In general, however, application to the locus to be protected of from 0.01 to 10.0kg per hectare of the compound of Formula I will be satisfactory.

For application, the compound generally is applied most effectively by formulating it with a suitable inert carrier or surface-active agent, or both. The invention, therefore, also includes compositions suitable for combating unwanted plants, such compositions comprising an inert carrier or surface-active agent, or both, in association with, as active ingredient at least one compound of Formula I, and a method of making such a composition which comprises bringing a compound of formula I into association with at least one carrier.

The term "carrier" as used herein means an inert solid or fluid material, which may be inorganic or organic and of synthetic or natural origin, with which the active compound is mixed or formulated to facilitate its application to the plant, seed, soil or other object to be treated, or its storage, transport and/or handling. Any of the materials customarily employed in formulating pesticides, herbicides, or fungicides, are suitable. A carrier in a composition according to the invention is any material with which the active ingredient is

formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain

½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension conentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The preparation, isolation and physical properties of typical individual species of the compounds of Formula I, in particular instances, are described in the following examples. In each case, the identity of the product, and each of any intermediates involved, was confirmed by appropriate chemical and spectral analyses.

Example 1

2-(1,1-Dimethyl-2-propynyl)-5-(3-methyl-2,4,5-trioxo-imidazolidin-1-yl)-1,3,4-thiadiazole (1)

A flask was charged with 456.3g of zinc and about 500ml of a mixture of 1050ml of benzene and 600ml of ether. About 2g of mercuric chloride was added and the resulting mixture was heated at 64°C and stirred slowly while a solution of 1369g of ethyl 2-bromoisobutanoate in 716g of acetic anhydride was added, drop by drop at such a rate as to maintain the temperature of the reaction mixture at 64°C. The addition required about 1.5 hours. Then the mixture was stirred at 65°C for 3 hours, held at room temperature for 18 hours and poured into 2 litres of ice water containing 250ml of concentrated hydrochloric acid. The organic phase was separated from the aqueous phase, dried ($MgSO_4$) and filtered. The filtrate was concentrated at 40°C under reduced pressure (30 Torr.) to give ethyl 2,2-dimethyl-3-oxobutanoate (1A), as an amber liquid.

A flask was charged with 1200.9g of 1A, 290ml of phosphorus oxychloride and 10ml of dimethylformamide. This mixture was stirred at 55°C, and 1751.4g of phosphorus trichloride was added, in portions, at such a rate as to maintain an internal reaction temperature of 55°C with low heat applied via a heating mantle. Then the mixture was stirred at 55-60°C for 5 hours, held at room temperature for 60 hours, and concentrated at 30-35°C and 30 Torr. for several hours. The residue was poured into ice water, the mixture was stirred for 45 minutes, and extracted with methylene chloride. The extract was dried ($MgSO_4$), filtered and concentrated to give crude (73%) ethyl 2,2-dimethyl-3-chloro-3-butenoate (1B).

A flask was charged with 2 litres of water and 592g of sodium hydroxide. The entire crude product, containing 959g of 1B, was added, followed by addition of 500ml of methanol. The resulting mixture was stirred at reflux temperature for 3 hours, then over a period of 2 hours, distillate was removed, and the remainder was steam-distilled for 2 hours. The aqueous residue was poured over ice and made distinctly acid with concentrated

hydrochloric acid. The resulting mixture was extracted with methylene chloride, and the extract was dried ($MgSO_4$) and concentrated to give 2,2-dimethyl-3-chloro-3-butenoic acid (1C), as an amber syrup.

A solution of 588g of 1C in 300ml of methylene chloride was added drop by drop over 15 mintes to a mixture of 521.2g of thionyl chloride and 5ml of dimethylformamide at room temperature. The mixture was heated at reflux for 3 hours, then distilled under reduced pressure to give 2,2-dimethyl-3-chloro-3-butyryl chloride (1D), b.p.: 95-99°C/-70 Torr.

336g of 1D was added drop by drop to a stirred mixture of 202g of dry ether, 76.8g of methanol and 202g of triethylamine, the rate of addition being such as to maintain a gentle reflux of the mixture, and requiring about 30 minutes. The mixture then was protected from atmospheric moisture, held at room temperature for 2 days, and filtered. The filtrate was concentrated to give methyl 2,2-dimethyl-3-chloro-3-butenoate (1E), as an amber liquid.

A solution of 200g of sodium hydroxide in 200ml of water was added to a stirred mixture of 327g of 1E and 500ml of dimethyl sulfoxide. The resulting mixture was stirred at 100°C for 24 hours, poured into ice water and made distinctly acidic with concentrated hydrochloric acid. The mixture was saturated with ammonium chloride and extracted with ether. The extract was dried ($MgSO_4$), concentrated, and the residue was distilled at reduced pressure to give 2,2-dimethyl-3-butynoic acid (1F), as a colourless liquid.

67.3g of phosphorus oxychloride was added drop by drop to a stirred mixture of 45.0g of 1F, 36.4g of thiosemicarbazide and 250ml of p-dioxane, at 90°C. The addition required about 30 minutes. The temperature was allowed to reach 96°C (reflux) during the addition, and after the addition was complete, the mixture was stirred and refluxed for 3.5 hours. The mixture then was cooled to room temperature and decanted. The dioxane

layer was treated with ice water and made alkaline with concentrated ammonium hydroxide. The resulting mixture was filtered and the solid was recrystallized from hot ethanol to give 2-amino-5-(1,1-dimethyl-2-propynyl)-1,3,4-thiadiazole (1G), as a light tan solid, m.p.: 172-173°C.

A solution of 13.0g of 1G and 4.45g of methyl isocyanate in 175ml of tetrahydrofuran was refluxed for 1.5 hours, and held overnight at room temperature. Then the solvent was evaporated, and the residue was recrystallized from ether-hexane to give N-(5-(1,1-dimethyl-2-propynyl)-1,3,4-thiadiazol-2-yl)-N'-methylurea (1H), as a white solid, m.p.: 181-184°C.

1.3g of oxalyl chloride was added to a solution of 2.24g of 1H in 100ml of toluene. The mixture was refluxed for 30 minutes, cooled to 0°C and filtered, to give 1, as a white solid, m.p.: 192-193°C.

Example 2

2-(1,1-Dimethyl-2-propynyl)-5-(4-hydroxy-1-methyl-2-oxoimidazolidin-3-yl)-1,3,4-thiadiazole (2)

A solution of 13g of 1G in 50ml of ethyl acetate was added drop by drop to a solution of 4.3g of hydrogen chloride and 15.45g of phosgene in 100ml of ethyl acetate, at 10-15°C. The resulting mixture was refluxed (67°C) for 2 hours, then was concentrated in a rotary evaporator to give 5-(1,1-dimethyl-2-propynyl)-1,3,4-thiadiazol-2-yl isocyanate dimer (2A), as a white solid, m.p.: 237°C (with decomposition).

A solution of 5.0g of 2A and 3.6g of N-(methylamino)-acetaldehyde dimethyl acetal in 125ml of tetrahydrofuran was refluxed for 1 hour, then concentrated in a rotary evaporator. Recrystallization of the residue from ether-hexane gave N-(5-(1,1-dimethyl-2-propynyl)-1,3,4-thiadiazol-2-yl)-N'-methyl-N'-(2,2-dimethoxyethyl)urea (2B), as a beige solid, m.p.: 71°C.

A mixture of 5.0g of 2B, 10ml of concentrated hydrochloric acid and 1300ml of water was stirred and refluxed for 30 minutes. The mixture then was cooled and extracted with

methylene chloride. The extract was dried ($MgSO_4$) and concentrated. The residue was triturated with hexane, cooled in a dry ice/acetone bath and filtered. The resulting solid was dried to give 2, m.p.: 45°C.

Example 3

2-(1,1-Dimethyl-2-propynyl)-5-(4-hydroxy-1-cyclopropyl-2-oxoimidazolidin-3-yl)-1,3,4-thiadiazole (3)

3 was prepared, as a lavender solid, m.p.: 125°C, from N-(cyclopropylamino)acetaldehyde dimethyl acetal, by the method described in Example 2.

Example 4

2-(1,1-dimethyl-2-propynyl)-5-(4,5-dihydroxy-3-methyl-2-oxoimidazolidin-1-yl)-1,3,4-thiadiazole (4)

11.6g of a 40% aqueous solution of glyoxal was added drop by drop to a stirred refluxing solution of 4.5g of 1H in 200ml of toluene. The mixture then was refluxed for 5 hours, water being removed therefrom azeotropically. The resulting mixture was concentrated under reduced pressure and the residue was crystallized from ether-hexane to give 4, as an off-white solid, m.p.: 78°C.

Example 5

2-(1,1-dimethyl-2-propynyl)-5-(5-hydroxy-1-methyl-2,4-dioxoimidazolidin-2,4-dione-3-yl)-1,3,4-thiadiazole (5)

2-(1,1-dimethyl-2-propynyl)-5-(5-hydroxy-3-methyl-2,4-dioxomidazol-2,4-dione-1-yl)-1,3,4-thiadiazole (6)

A solution of 4.6g of glyoxylic acid hydrate in 25ml of tetrahydrofuran was added to a solution of 5.6g of 1H in 150ml of methylene chloride, at 30°C. The mixture was refluxed for 3 hours, then concentrated under reduced pressure. The residue was chromatographed over silica gel using a 1:1:2 v:v:v mixture of tetrahydrofuran, ethyl acetate and hexane as eluent. Workup of the eluted materials gave 6, a viscous liquid, as the major product, and 5 as a colourless solid, m.p.: 155-157°C.

Example 6

Herbicidal Activity Plants

In the following examples, the species of plants that were tested were:

Barnyardgrass (watergrass) - *Echinochloa crus-galli*
Large crabgrass - *Digitaria sanguinalis*
Downy brome - *Bromus tectorum*
Yellow foxtail - *Setaria lutescens*
Redroot pigweed - *Amaranthus retroflexus*
Sicklepod - *Cassia obtusifolia*
Velvetleaf - *Abutilon theophrasti*
Garden cress - *Lepidium sativum*
Johnsongrass - *Sorghum halepense*

Test Procedures

The preemergence (soil) herbicidal activity of the compounds was evaluated by planting seeds of barnyardgrass, garden cress, downy brome, velvetleaf, yellow foxtail, and sicklepod in test tubes, nominally measuring 25 x 200 millimeters, filled about three-quarters full of untreated soil, in each case covered on top with about 2.5 cubic centimeters of soil treated with a certain amount of the test compund. The treated soil applied to the tubes containing the barnyardgrass and cress seeds contained one milligram of the test compound per tube, and contained 0.1 milligram of the test compound per tube containing the seeds of each of the other plants. The dosages were approximately 20 and 2.0 kg of test compound per hectare, respectively. The seeds were planted on top of the treated soil and covered with about 1.5 cubic centimeters of untreated soil. The planted soil was held under controlled conditions of temperature, moisture, and light for 9 to 10 days. The amounts of germination and growth in each tube were evaluated on a 0 to 9 scale, the numeric ratings having the following meanings:

| Rating | Meanings |
|---|---|
| 9 | No living tissue |
| 8 | Plant severely damaged and expected to die |
| 7 | Plant badly damaged, but expected to live |

| | |
|---|---|
| 6 | Moderate damage, but complete recovery expected |
| 5 | Intermediate damage (probably unacceptable for crop plants) |
| 3-4 | Observable damage |
| 1-2 | Plant slightly affected, possibly by the chemical, possibly due to biological variability |
| 0 | No visible effect |

The postemergence (foliar) herbicidal activity of compounds of the invention was evaluated by spraying 10-day-old large downy brome plants in some cases, 6-day-old Johnsongrass plants in other cases, 9-day-old velvetleaf plants, 9-day-old yellow foxtail plants and 9-day-old sicklepod plants to runoff with a liquid formulation of the test compound. The crabgrass and pigweed plants were sprayed with 2.4 milliliters of a 0.25% solution (about ten kg of the test compound per hectare), and other plants were sprayed with 2.4 millilitres of a 0.025% solution (about one kg of the test compound per hectare). The sprayed plants were held under controlled conditions of temperature, moisture and light for 7 to 8 days, and the effect of the test compound was then evaluated visually, the results being rated on the 0 to 9 scale described above.

Results of the preemergence and postemergence herbicidal activity tests are set forth in Table I.

Table I - HERBICIDAL ACTIVITY

| | Preemergence | | | | | | Postemergence | | | | | |
|---|---|---|---|---|---|---|---|---|---|---|---|---|
| Compound | Barnyard-grass | Garden Cress | Downy Brome | Velvet-leaf | Yellow Foxtail | Sickle pod | Crab-grass | Pig-weed | Johnson-grass | Velvet-leaf | Yellow Foxtail | Sickle pod |
| 1 | 9 | 9 | 8 | 9 | 9 | 9 | 9 | 9 | 3 | 4 | 4 | 6 |
| 2 | 8 | 8 | 7 | 9 | 7 | 9 | 9 | 9 | 8 | 9 | 9 | 9 |
| 3 | 9 | 9 | 9 | 9 | 9 | 9 | 9 | 9 | 2 | 5 | 3 | 2 |
| 4 | 9 | 9 | 5 | 9 | 7 | 8 | 7 | 9 | 2 | 6 | 5 | 4 |
| 5 | 9 | 9 | 2 | 9 | 6 | 9 | 8 | 9 | 4 | 3 | 4 | 5 |
| 6 | 9 | 9 | 2 | 8 | 3 | 8 | 5 | 7 | 2 | 2 | 2 | 3 |

Example 7

Herbicidal Selectivity

In the following examples, the species of plants that were tested were:

Barnyardgrass
Downy brome
Johnsongrass
Wild oats - Avena fatua
Yellow foxtail
Goose grass - Eluesine indica L.
Yellow nutsedge - Cyperus esculentus L.
Morning glory - Ipomoea purpurea L. (Roth)
Wild muxtard - Brassica kaber
Redroot pigweed
Sickelpod
Velvetleaf
Corn - Zea mays
Cotton - Gossypium hirsutum
Rice - Oryza sativa
Grain sorghum - Sorghum vulgare
Soybeans - Glycine max
Sugarbeets - Beta vulgaris
Wheat - Triticum aestivum
Nightshade - (Solanum nigrum sp.)

Test Procedures

The preemergence activity of Compound 2 was further determined with respect to certain species of crop plants and common species of weeds, by spraying a formulation of the compound on soil in small pots in which seeds of the plants had been sown. The postemergence herbicidal activity of Compound 2 was evaluated with respect to the crop plants and weeds, by spraying a formulation of the compound on the foliage of the young growing plants. In each series of tests, the plants were grown in narrow trays and sprayed with the formulation. The results of the tests were evaluated on the basis of the 0-9

scale described with respect to the earlier tests. The results of the tests are reported in Table II.

Table II - COMPOUND 2

| Plant Species | Rating of Effect at Indicated Dosage (kg/ha) | | | | |
|---|---|---|---|---|---|
| | Preemergence | | Postemergence | | |
| | 1/8 | 1/32 | 1/4 | 1/8 | 1/32 |
| Corn | 0 | 0 | 6 | 5 | 3 |
| Cotton | 4 | 0 | 8 | 8 | 7 |
| Rice | 8 | 3 | 0 | 0 | 0 |
| Grain Sorghum | 7 | 0 | 4 | 4 | 2 |
| Soybean | 0 | 0 | 9 | 9 | 8 |
| Sugar Beet | 4 | - | 9 | 9 | 7 |
| Wheat | 9 | 0 | 3 | 2 | 0 |
| Barnyard Grass | - | - | 5 | 4 | 2 |
| Downy Brome | 5 | 0 | 2 | 0 | 0 |
| Johnsongrass | 9 | 0 | 6 | 3 | 0 |
| Wild Oats | 9 | 0 | 5 | 4 | 0 |
| Yellow Foxtail | 9 | 0 | 5 | 4 | 2 |
| Goose Grass | 5 | 0 | 6 | 5 | 2 |
| Yellow Nutsedge | - | - | 3 | 2 | 0 |
| Morning Glory | 0 | 0 | 9 | 9 | 5 |
| Mustard | 9 | 8 | 8 | 8 | 5 |
| Pigweed | 8 | 6 | 7 | 7 | 5 |
| Sicklepod | 9 | 2 | 9 | 9 | 8 |
| Velvetleaf | 9 | 3 | 8 | 7 | 5 |
| Nightshade | - | - | 9 | 9 | 7 |
| Cocklebur | - | - | 9 | 7 | 4 |

K 3433

CLAIMS

1. Compounds of the general formula:

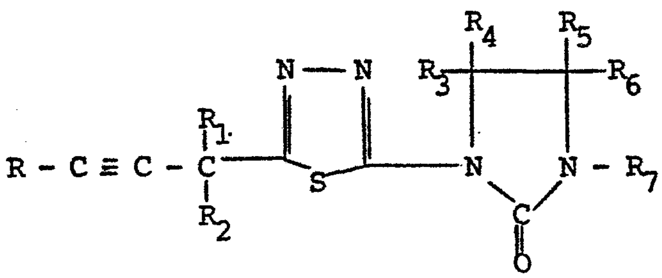

wherein R, $R_1$, and $R_2$, which may be the same or different, each independently represents a hydrogen atom or a lower alkyl group; $R_3$ represents a hydrogen atom and $R_4$ represents a hydroxyl group; or $R_3$ and $R_4$ together jointly represent an oxo (=O) group; $R_5$ is a hydrogen atom and $R_6$ is a hydrogen atom or a hydroxy group, or $R_5$ and $R_6$ together jointly represent an oxo group; and $R_7$ is a lower alkyl or alkoxy group or a cyclopropyl group.

2. Compounds as claimed in claim 1 wherein R is a hydrogen atom or a methyl group; $R_1$ is methyl or ethyl; $R_2$ is a hydrogen atom or a methyl or ethyl group; and $R_7$ is an alkyl or alkoxy group of 1 to 3 carbon atoms or a cyclopropyl group.

3. Compounds as claimed in claim 2 wherein R represents a hydrogen atom and each of $R_1$, $R_2$ and $R_7$ represents a methyl group.

4. Compounds as claimed in claim 1, 2 or 3 wherein each of $R_3$, $R_5$ and $R_6$ represents a hydrogen atom and $R_4$ represents a hydroxyl group.
5. Process for the preparation of a compound as defined in claim 1, which comprises reacting a urea of formula III:-

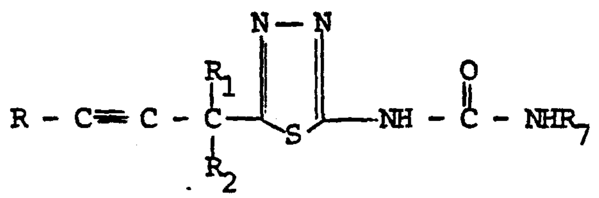

(III)

with a diketo compound of the formula X-CO-CO-X, wherein both groups X represent a halogen atom, or one X represents a hydrogen atom and the other represents a hydrogen atom or a hydroxy group.
6. Process as claimed in claim 5 wherein the reaction is effected in an inert solvent at elevated temperature.
7. A compound as defined in claim 1, whenever prepared by a process as claimed in claim 5 or 6.
8. Herbicidal composition, which comprises a compound as claimed in any of claims 1 to 4 and 7, together with a carrier.
9. A composition as claimed in claim 8, which comprises at least two carriers, at least one of which is a surface-active agnet.
10. Method of combating undesired plant growth at a locus, which comprises treating the locus with a compound as claimed in any one of claims 1 to 4 and 7, or a composition as claimed in either of claims 8 or 9.
11. The use of a compound as claimed in any one of claims 1 to 4, and 7 as herbicide.

European Patent Office

# EUROPEAN SEARCH REPORT

0136754

Application number

EP 84201271.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 314 842 (LAVANISH)<br>* Claims 1,7 *<br>-- | 1,8 | C 07 D 417/04<br>A 01 N 43/82//<br>(C 07 D 417/04<br>C 07 D 285:08<br>C 07 D 233:32) |
| A | US - A - 4 043 795 (KRENZER)<br>* Formula II; claim 9 *<br>-- | 1,8 | |
| A | US - A - 4 023 957 (KRENZER)<br>* Claims 1,2 *<br>-- | 1,8 | |
| A | US - A - 4 063 924 (KRENZER)<br>* Abstract; example 3 *<br>-- | 1,8 | |
| A | US - A - 4 086 238 (KRENZER)<br>* Abstract; columns 16,17 *<br>-- | 1,8 | TECHNICAL FIELDS SEARCHED (Int. Cl.4) |
| A | US - A - 3 984 228 (KRENZER)<br>* Abstract *<br>-- | 1,8 | C 07 D 417/00 |
| A | DE - A1 - 3 030 661 (BAYER)<br>* Formula Ib; example II/5 *<br>-- | 1,8 | |

The present search report has been drawn up for all claims

| Place of search | Date of completion of the search | Examiner |
|---|---|---|
| VIENNA | 16-11-1984 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

0136754
Application number

EP 84201271.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 94, no. 21, May 25, 1981, Columbus, Ohio, USA<br>ASKARI, SYED H; MOSS, STEPHEN F; TAYLOR, DAVID R. "Thiadiazoles and thiadiazolines. Part 1. Reaction of thiourea and ethylenethiourea with chlorodiazabutadienes: a new route to 4-amidino-1,3,4-thiadiazolines"<br>page 720, column 1, abstract-no. 174 996k<br>& J. Chem. Soc., Perkin Trans. 1 1981, (2), 360-5<br>-- | 1 | |
| A | CHEMICAL ABSTRACTS, vol. 89, no. 13, September 25, 1978, Columbus, Ohio, USA<br>MARTVON, A; FLOCH L; SEKRETAR S. "Reactions of N,N'-thiocarbonyl diimidazole with 1,3-dipolar agents. The synthesis of 5-substituted-2-(1-imidazolyl)-1,3,4-thiadiazoles and 5-(1-imidazolyl)-1,2,3,4-thiatriazole"<br>page 913, column 1, abstract-no. 109 388w<br>& Tetrahedron 1978, 34(4), 453-6<br>-- | 1 | TECHNICAL FIELDS SEARCHED (Int. Cl.4) |

The present search report has been drawn up for all claims

| Place of search | Date of completion of the search | Examiner |
|---|---|---|
| VIENNA | 16-11-1984 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

0136754
Application number

EP 84201271.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 87, no. 11, September 12, 1977, Columbus, Ohio, USA<br>FURNESS, W; HALAWI M.H; BARLOW J. N. "Properties of some imidazolidinones and trials with buthidazole and its derivatives"<br>page 138, column 2, abstract-no. 79 424u<br>& Proc. Br. Weed Control Conf. 1976, 13, vol. 2, 731-8<br>-- | 1,8 | |
| A | CHEMICAL ABSTRACTS, vol. 92, no. 13, March 31, 1980, Columbus, Ohio, USA<br>KUNKEL J.F; LIRA E.P. "Herbicidal activity of a substituted thiadiazolyl urea (VEL-3510) and of the acetate and octanoate esters of buthidazole"<br>page 205, column 1, abstract-no. 105 745z<br>& Int. Velsicol Symp., [Proc.] 1977, 11th Paper No. 7, 12pp.<br>-- | 1,8 | TECHNICAL FIELDS SEARCHED (Int. Cl.4) |

The present search report has been drawn up for all claims

| Place of search | Date of completion of the search | Examiner |
|---|---|---|
| VIENNA | 16-11-1984 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number EP 84201271.8

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 96, no. 13, March 29, 1982, Columbus, Ohio, USA<br>NOMURA, NORMAN S; HILTON, H. WAYNE "Distribution of residues and metabolism of the herbicide buthidazole in sugarcane from root and foliar treatments" page 263, column 2, abstract-no. 99 301r<br>& J. Agric. Food Chem. 1982, 30(2), 232-8<br>---- | 1,8 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 16-11-1984 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document